# EUROPÄISCHE PATENTANMELDUNG

(11) **EP 1 357 102 A1**
(43) Veröffentlichungstag der Anmeldung: **29.10.2003**
(21) Anmeldenummer: 03009277.9
(22) Anmeldetag: 24.04.2003
(51) Int. Cl.: C07C 41/50, C07B 41/04

(54) **Verfahren zur Herstellung von Acetalen und Ketalen mit Hilfe mehrstufiger Pervaporation oder Dampfpermeation**

(30) Priorität: 27.04.2002 DE 10218916
(71) Anmelder: Degussa AG, 40474 Düsseldorf (DE)
(72) Erfinder: Köhler, Günther, Dr., 45770 Marl (DE); Neumann, Manfred, Dr., 45770 Marl (DE); Poll, Heinz-Günter, Dr., 45770 Marl (DE); Bauer, Frank, Dr., 45721 Haltern am See (DE); Winkler, Hermann, 45772 Marl (DE)

(57) **Zusammenfassung**

Die Erfindung betrifft ein Verfahren zur Herstellung von Acetalen oder Ketalen aus Carbonylverbindungen und Alkoholen in Gegenwart von sauren Katalysatoren, wobei Wasser und Alkohol aus dem Reaktionsgemisch durch eine mindestens zweistufig durchgeführte Pervaporation oder Dampfpermeation entfernt werden und wobei zusätzlicher Alkohol in den Retentatstrom mindestens einer der Stufen eingespeist wird und mindestens eine der Membranen in den Pervaporations- oder Dampfpermeationsstufen von der oder den anderen Membranen in ihrem Selektivitätsverhalten bezüglich der Abtrennung von Wasser und Alkohol verschieden ist.

## Beschreibung

Die Erfindung betrifft ein Verfahren zur Herstellung von Acetalen und Ketalen aus Carbonylverbindungen und Alkoholen in Gegenwart von sauren Katalysatoren, wobei Wasser und Alkohole durch mehrstufige Pervaporation oder Dampfpermeation abgetrennt werden.

Verschiedene Acetale und Ketale sind wichtige Edukte, Zwischenverbindungen, Schutzgruppen oder Lösemittel in der organischen Chemie. Die Herstellung von Acetalen und Ketalen ist nach dem Stand der Technik durch Umsetzung von Carbonylverbindungen mit Alkoholen oder Orthocarbonsäureestern in Gegenwart von sauren Katalysatoren, die in homogener Lösung oder auch als feste saure Ionentauscher vorliegen können, möglich (siehe zum Beispiel B. Ullmann III, 15; Houben-Weyl VI/3,3, 204; VII/1, 418, 436; D. P. Roelofsen, H. van Bekkum, Synthesis 1972, 419).

In J. Org. Chem., 1959, 1731, beschreiben Lorette et al. den Einfluss der Eduktstöchiometrien und der Temperaturen auf die Kinetik dieser Umsetzung, insbesondere die Tatsache, dass das Gleichgewicht dieser Reaktion weitgehend auf der Seite der Edukte liegt und es deshalb unter moderaten Bedingungen nicht möglich ist, einen vollständigen Eduktumsatz zu erzielen. Wie weiterhin beschrieben wird, sind zur Erreichung guter Eduktumsätze und Raum-Zeit-Ausbeuten niedrige Temperaturen von bis zu - 20 °C notwendig. Dabei muss zum Beispiel bei der Herstellung von Dimethoxypropan ein Verhältnis von Aceton zu Methanol von 1 : 2 bis 1 : 4 eingestellt werden. Die Ketalisierung beschreiben Lorette et al. in Gegenwart von sauren Ionenaustauschern. Die Aufarbeitung des so erzeugten Gemisches, dessen Wassergehalt 3 - 4 Gew.-% beträgt, ist durch die Entstehung von Azeotropen zwischen Dimethoxypropan, Wasser und Methanol einerseits und Aceton und Methanol andererseits aufwändig, um die Ausbeute von Dimethoxypropan zu optimieren und die Verluste entsprechend niedrig zu halten.

In der Patentschrift US 2 827 495 wird die Aufarbeitung einer wässrig-methanolischen Leichtsiedermischung durch Extraktion mit wässriger Lauge, insbesondere Natronlauge, beschrieben. Durch dieses technisch als Gegenstromextraktion ausgeführte Verfahren kann als organisches Produkt der Extraktion ein methanolfreies, nahezu reines Dimethoxypropan erhalten werden. Nachteilig ist allerdings, dass im Reaktionsprodukt ein relativ hoher Wassergehalt enthalten ist.

In der Patentschrift US 1 850 836 von Guinot et al. wird die Herstellung und Isolierung von Acetalen beschrieben, nämlich die Umsetzung eines Aldehyds mit einem Alkohol in Gegenwart einer katalytischen Menge einer Mineralsäure, insbesondere von gasförmigem Chlorwasserstoff. Nach Erreichen der Gleichgewichtseinstellung der Reaktion wird mit einer der Säure mindestens äquivalenten Menge Base neutralisiert, um die Rückreaktion bei der Aufarbeitung zu unterdrücken, und anschließend durch Zugabe eines aliphatischen Hilfslösemittels, das wasserunlöslich ist und mit dem eingesetzten Alkohol ein Minimumazeotrop bildet, aufgearbeitet. Dabei wird Acetal mittels eines nicht mit Wasser mischbaren aliphatischen Lösemittels von Wasser und weitgehend vom Alkohol befreit und der Alkohol anschließend als Azeotrop mit dem Aliphaten destillativ abgetrennt. Es ist offensichtlich, dass die Anwesenheit von erheblichen Mengen an Aliphaten zur vollständigen Entfernung des Alkohols notwendig ist, wobei anschließend zur Abtrennung des Methanols vom aliphatischen Lösemittel eine wässrige Extraktion notwendig ist. Insgesamt ist das Verfahren kompliziert und wenig zur technischen Umsetzung geeignet.

In der Patentschrift US 2 837 575 wird eine Ketalisierung in Gegenwart von gasförmigem Chlorwasserstoff beschrieben. Zur Erhöhung des Acetonumsatzes werden bis zu 8 Gew.-% Chlorwasserstoff verwendet, der anschließend mit Natronlauge neutralisiert werden muss und einen nicht unerheblichen Salzanfall produziert. Die anschließende Aufarbeitung erfolgt durch aufwändige Extraktionen mit Natronlauge verschiedener Konzentrationen und zusätzliche Extraktion mit einem leichtflüchtigen aliphatischen Kohlenwasserstoff. Es ist dabei eine Vielzahl an Trennoperationen erforderlich, so dass sich der Prozess für eine technische Umsetzung aus wirtschaftlichen Gründen wenig eignet.

In der Patentschrift US 4 775 447 wird ein Verfahren zur Herstellung von Dimethoxypropan beschrieben, bei dem ein saurer heterogener Ionenaustauscher als Katalysator verwendet und das Verhältnis von Aceton : Methanol zwischen 1 : 1 und 1 : 3 eingestellt wird. Die Aufarbeitung ist jedoch außerordentlich kompliziert und umfasst zunächst die destillative Entfernung eines acetonreichen Azeotrops bestehend aus Aceton, Wasser und Methanol. Zum restlichen Gemisch Methanol-Dimethoxypropan muss eine entsprechende Menge an Aceton dosiert werden, so dass in einer zweiten Destillation ein Azeotrop der Zusammensetzung Aceton-Methanol (ca. 86 Vol.-% Aceton - 14 Vol.-% Methanol) entfernt wird. Auf die Trennung Aceton-Methanol wird in der Patentschrift nicht näher eingegangen. Die Abtrennung des durch die Reaktion gebildeten Reaktionswassers vom Dimethoxypropan wird nicht beschrieben.

Die destillative Trennung des komplexen Azeotropes im System Wasser-Dimethoxypropan-Aceton-Methanol werden von Brunner und Scholz in Chem.-Ing.-Tech. 52 (1980), 164 - 166 sowie in Khim. Farm. Zhl. 17 (1983), 454 - 459, beschrieben.
Brunner und Scholz revidieren die früheren Ergebnisse von Lorette et al. über die Existenz eines ternären Azeotrops zwischen Aceton, Methanol und Dimethoxypropan und kommen zu dem Ergebnis, dass ein acetonreiches Azeotrop (Zusammensetzung wie oben) zwischen Aceton und Methanol mit einem Siedepunkt von 55,4 °C und ein weiteres Azeotrop zwischen Methanol und Dimethoxypropan mit einem Siedepunkt von 61,0 °C mit der Zusammensetzung 72,5 Mol-% Methanol und 27,5 Mol-% Dimethoxypropan existiert.

Auch die Herstellung von ungesättigten Ethern aus den entsprechenden Acetalen beziehungsweise Ketalen ist in der Literatur beschrieben. In der Patentschrift US 2 667 517 wird die Eliminierung von Alkohol in Gegenwart eines sauren Katalysators aus der Gruppe der Sulfonsäuren in einem Kohlenwasserstoff oder einem Chlorkohlenwasserstoff als Lösebeziehungsweise Verdünnungs-Mittel beschrieben. Probleme entstehen bei dieser Verfahrensweise durch bei der Reaktion sich bildende Hochsieder.

In EP-A-0 197 283 wird die Verwendung eines Mineralöls, dass nach Gebrauch verbrannt wird und dadurch einen nicht unerheblichen spezifischen Verbrauch des Katalysators verursacht, beschrieben. Es ist auch möglich, in Abwesenheit eines Lösemittels zu arbeiten, wie in DE-A-40 39 950 vorgeschlagen wird. Dabei wird das Ketal in Gegenwart eines Katalysatorsystems, bestehend aus speziellen Säuren, bei erhöhten Temperaturen (bis 200 °C) generiert. Das Verfahren ist nicht allgemein anwendbar und hat den Nachteil, dass ebenfalls Verunreinigungen der Katalysatorphase durch unselektiv verlaufende Reaktionen beziehungsweise thermische Instabilitäten der eingesetzten Substanzen auftreten, die eine Ausschleusung notwendig machen.

Allen diesen Verfahren ist gemeinsam, dass die Isolierung des Produktgemisches aufgrund der entstehenden Azeotrope mit Methanol und Wasser beziehungsweise Methanol und Dimethoxypropan bei der destillativen oder extraktiven Aufarbeitung sehr aufwändig ist und es darüber hinaus auch zur Rückreaktion zum Beispiel der Ketale mit Wasser kommen kann, wenn keine entsprechenden Maßnahmen getroffen werden.
Darüber hinaus sind Umsatz und Ausbeute vor allem bei solchen Verfahren begrenzt - wie in EP-A-1 167 333 beschrieben -, wenn mit Hilfe der Pervaporation an unselektiven Membrantypen neben Wasser große Mengen an Alkohol gleichzeitig abgetrennt werden, so dass rein stöchiometrisch ein Unterschuss an einem der Reaktionspartner entsteht. In diesen Fällen stagniert selbst bei Anwendung mehrerer Membranstufen der Umsatz bei ca. 55 - 60 %. Eine aufwändige Aufarbeitung des Produktgemisches zur Erzielung hoher Reinproduktqualitäten ist dananch unerlässlich.

Es bestand daher die Aufgabe, ein verbessertes Verfahren zu finden, das es ermöglicht, besonders hohe Reinheiten der Reaktionsprodukte zu erzielen und große Raum-Zeit-Ausbeuten zu verwirklichen, wobei möglichst wenige Aufarbeitungsstufen nachgeschaltet werden sollen.

Die Aufgabe wird erfindungsgemäß dadurch gelöst, dass man die Acetalisierung beziehungsweise Ketalisierung in einem Reaktor, der gleichzeitig auch Verdampfer sein kann, durchführt, wobei man die Carbonylverbindung absatzweise oder kontinuierlich mit einem Alkohol in Gegenwart eines sauren Katalysators zusammenführt und das bei der Reaktion anfallende Wasser und den Alkohol aus dem Reaktionsgemisch durch eine mindestens zweistufig durchgeführte Pervaporation oder Dampfpermeation entfernt, wobei zusätzlicher Alkohol in mindestens einer der Stufen als Zufuhrstrom (feed) zur nächsten Stufe eingespeist wird und mindestens eine der Membranen in den Pervaporations- oder Dampfpermeationsstufen von den anderen Membranen in ihrem Selektivitätsverhalten bezüglich der Abtrennung von Wasser und Alkohol verschieden ist.

Die zusätzlich eingespeiste Menge an Alkohol beträgt vorzugsweise 10 - 500 Mol-%, bezogen auf die eingesetzte Carbonylverbindung.

Wasser soll dabei bevorzugt durch die Anwendung möglichst selektiv arbeitender Membrantypen abgetrennt werden, um das Reaktionsgleichgewicht auf die Seite der Produkte zu verschieben, wodurch der Reaktionsfortschritt beschleunigt wird. In einer weiteren Stufe, die sich als kontinuierlicher oder absatzweiser geführter Verfahrensschritt anschließt, werden erneut und ergänzend weitere Alkoholmengen eingespeist, um den Umsatz mit der Carbonylverbindung zu maximieren. Anschließend wird in einer abschließenden Membranstufe der Alkohol vom Acetal beziehungsweise Ketal abgetrennt, so dass ein sehr reines Produkt ohne jede weitere Nachbehandlung anfällt.

Als Carbonylverbindungen werden Aldehyde der allgemeinen Formel RCHO oder Ketone der allgemeinen Formel R¹COR² eingesetzt, wobei R, R¹ und R² gleich oder verschieden sind und für einen Alkyl-, Alkenyl-, Cycloalkyl-, Aryl-, Alkylaryl- oder Aralkyl-Rest mit 1 - 15 Kohlenstoffatomen stehen und R¹ und R² auch zu einem Ring verbunden sein können, und wobei der Alkylrest auch eine Acylfunktion mit 1 bis 8, vorzugsweise 1 bis 6, Kohlenstoffatomen tragen kann.

Insbesondere seien als Carbonylverbindungen Formaldehyd, Acetaldehyd, Propionaldehyd, n-und iso-Butyraldehyd, Acrolein, Benzaldehyd, Formamid, N,N-Dimethylformamid, Aceton, Methylethylketon, Acetophenon, Cyclohexanon, Cyclododecanon, Cyclodecandion und Acetessigsäureester genannt.

Als Alkohole werden ein- oder mehrwertige (vorzugsweise zweiwertige), unverzweigte oder verzweigte, gesättigte, ungesättigte oder aromatische Alkohole mit 1 bis 10, vorzugsweise mit 1 bis 3 Kohlenstoffatomen, eingesetzt. Insbesondere seien als Alkohole Methanol, Ethanol, n-Propanol, iso-Propanol, Allylalkohol, Benzylalkohol, Ethylenglykol, Propandiol-1,3 und Propandiol-1,2 genannt.

Die Herstellung betrifft insbesondere Formaldehyddimethylacetal, Formaldehyddiethylacetal, Acetaldehyddimethylacetal, Acetaldehyddiethylacetal, 2,2-Dimethoxypropan, 2,2-Diethoxypropan, Dimethoxycyclohexan, Diethoxycyclohexan, Dimethoxycyclooctan, Diethoxycyclooctan, Dimethoxycyclododecan oder Diethoxycyclododecan.
Als saure Katalysatoren eignen sich saure Ionentauscher wie zum Beispiel LEWATITE™ der Bayer AG, saure Montmorillonite, anorganische Säuren, wie zum Beispiel Schwefelsäure, Phosphorsäure und Borsäure, oder vorzugsweise organische Carbonsäuren mit 1 bis 3 Carboxylgruppen mit 1 bis 12, vorzugsweise 2 bis 10, Kohlenstoffatomen, wie zum Beispiel Ameisensäure, Essigsäure, Oxalsäure und 2-Ethylhexansäure, und weiterhin zum Beispiel nach dem Koch-Verfahren herstellbare verzweigte organische Säuren, wie zum Beispiel Neooctan-, Neononan- oder Neodecansäure, und die Gruppe der Sulfonsäuren wie zum Beispiel Methansulfonsäure oder p-Toluolsulfonsäure. Die Säuren können gelöst oder in einem Nebenstrom oder im Eduktgemisch suspendiert, oder aber auch als Feststoff - wie im Fall der Ionenaustauscher oder geträgerter saurer Katalysatoren - im Inneren des Reaktors als Packung fixiert vorliegen.

Das erfindungsgemäße Verfahren wird bei Temperaturen im Reaktor beziehungsweise auf der Retentatseite von - 10 bis + 200 °C, bevorzugt bei + 20 bis + 150 °C durchgeführt. Auf der Zufuhr-Seite wird ein Druck von 0,1 bis 10 bar, bevorzugt 0,5 bis 5 bar, angewendet. Auf der Permeatseite wird ein Druck von 0,1 bis 1000 mbar, bevorzugt 10 - 30 mbar eingestellt. Bei der Durchführung des erfindungsgemäßen Verfahrens wird das Zufuhr-Gemisch in flüssiger (Pervaporation) oder dampfförmiger Form (Dampfpermeation) an die Membran herangeführt. Druck und Temperatur auf der Zufuhr-Seite werden so eingestellt, dass der Druck nahe am Siededruck liegt.
Der Reaktor kann prinzipiell ein Kessel beziehungsweise ein Puffergefäß sein. Die Dämpfe oder die Flüssigkeit, bestehend aus Carbonylverbindung, Wasser, Alkohol und in Abhängigkeit vom Reaktionsfortschritt auch das Acetal beziehungsweise Ketal werden direkt mit Hilfe von Fördervorrichtungen der Membrananlage zugeführt, wobei jeweils eine Komponente möglichst selektiv aus diesem Zufuhrstrom abgetrennt wird. Der Zufuhrstrom kann alternativ aus dem Reaktor über einen separaten Verdampfer dem Membranmodul zugeführt werden, wobei im Membranmodul die entsprechende Komponente - Wasser oder/und Alkohol - über die Membran entfernt und das abgereicherte Retentat als Dampf oder nach Kondensation flüssig wieder zurückgeführt wird.

Im Falle der Pervaporation muss, da die abgetrennte Komponente im Permeat dampfförmig anfällt, die Verdampfungsenergie vom System aufgebracht werden, wobei sich das Retentat entsprechend abkühlt. Da der Stofftransport durch die Membran zudem temperaturabhängig ist beziehungsweise verbrauchte Wärme in Folge von Verdampfung ergänzt werden muss, wird der Zufuhr-Strom vorzugsweise über Vorwärmer aufgeheizt, um einen gleichbleibend optimalen Permeatfluss zu gewährleisten. Die abgetrennte Stoffmenge wird anschließend kondensiert oder flüssig wiedergewonnen. Die eingesetzte Vakuumpumpe dient in erster Linie dazu, das Vakuum konstant zu halten beziehungsweise Inertgase abzuführen.

Als Membranen können sowohl organische Polymere als auch anorganische Materialien - vorzugsweise auf keramischer Basis - verwendet werden. Polymermembranen sind im Prinzip bekannt und werden vorwiegend zur Entwässerung aber auch zur Abtrennung von Alkoholen aus Gemischen bestehend aus Kohlenwasserstoffen mit höher Kohlenstoffzahl eingesetzt, wie zum Beispiel in EP-A-0 629 600 beschrieben wird. Neuentwicklungen sind anorganische Membranen, die sowohl für Entwässerungsprozesse als auch für Trennungen von organischen Verbindungen eingesetzt werden. Diese Keramikmembranen besitzen vorzugsweise eine selektive, defektfreie Zeolithschicht, die aufgrund ihrer gleichmäßigen Kristallstruktur selektive Trenneigenschaften besitzt.
Über das Membranmodul werden Alkohol und Wasser in unterschiedlich arbeitenden Membrantypen selektiv von der Carbonylverbindung abgetrennt. Das entsprechend abgereicherte Retentat wird kondensiert und kann in den Reaktor und das Membranmodul zurückgeführt werden, wo es bei diskontinuierlichem oder kontinuierlichem Betrieb im Sumpf abgezogen werden kann. Gemäß vorliegender Erfindung wird das Gemisch einer weiteren Reaktorstufe zugeführt, in der der gleiche Alkohol wie als Edukt der ersten Stufe eingespeist wird, wodurch der Umsatz der Carbonylverbindung vervollständigt wird. Der als Permeat zusammen mit dem Wasser abgetrennte Alkohol kann bedarfsweise jedoch mit Hilfe einer einfachen Destillationsvorrichtung vom Wasser abgetrennt und als Edukt in die Reaktion zurückgeführt werden.
In bevorzugter Form werden Mehrschichtmembranen eingesetzt, das heißt, derartige Membranen bestehen aus einer Trägerschicht, einer porösen Stützschicht und einer eigentlichen Trennschicht. Als Trägerschicht kommen flexible Gewebe aus Fasern einschließlich Metallgeweben, Polyestern, Polyetheramiden und Kohlefasern usw. wie zum Beispiel Membranen des Typs PERVAP der Fa. SULZER-CHEMTECH oder NaA Zeolith Membranen der Fa. MITSUI in Frage. Gleichfalls geeignet sind anorganische Materialien mit porösen Strukturen wie zum Beispiel Keramik.
Die Pervaporation wird erfindungsgemäß mehrstufig ausgeführt. In einer ersten Stufe werden über den Reaktor, der mit einer Bypassleitung versehen ist, in der das Membranmodul zur Wasserabtrennung eingebunden ist, die Carbonylverbindung und der entsprechende Alkohol zur Umsetzung gebracht. Dieser Schritt kann absatzweise, zweckmäßigerweise aber kontinuierlich erfolgen. Bei kontinuierlicher Fahrweise werden soviel Carbonylverbindung und Alkohol als Zufuhrstrom über die Pumpen P1 und P2 (siehe Abbildung) dosiert, wie Gesamtmenge an Produkt über P4 und die an den Membranmodulen 1, 2 und 3 entfernte Wasser- und Alkoholmenge ausgetragen werden. In diesen Stufen kommen Membrantypen zum Einsatz, die große Wassermengen abtrennen - also einen hohen Fluss ermöglichen. Eine Reihe von Membrantypen arbeitet bei hohen Flüssen jedoch weniger selektiv, so dass auch mehr oder weniger große Mengen Alkohol mit abgetrennt werden.
In der beschriebenen Kaskadenfahrweise bewegt sich der Umsatz an Carbonylverbindung auf sehr hohem Niveau, wodurch in der letzten Membranstufe die Abtrennung des im Überschuss eingesetzten Alkohols vom Acetal beziehungsweise Ketal erforderlich ist. In dieser letzten Kaskadestufe wird ein Membrantyp eingesetzt, der große Stoffströme an Alkohol erlaubt, jedoch die hydrophoben Acetale beziehungsweise Ketale selektiv zurückhält (zum Beispiel NaY-Zeolith-Membranen). Um in der letzten Stufe die Rückreaktion des Ketals beziehungsweise Acetals unter Abspaltung von Alkohol zu vermeiden, kann die Katalysatorsäure zweckmäßigerweise als höhersiedender Sumpf in einer bei niedrigen Temperaturen und kurzen Verweilzeiten ausgeführten schonenden Verdampfung über einen Fallfilm- oder Dünnschicht-verdampfer bei niedrigen Temperaturen abgetrennt werden. Der abgetrennte Alkohol kann ohne weiteres in der Synthese zur Bildung des Acetals beziehungsweise Ketals wiederverwendet werden.

Das erfindungsgemäße Verfahren bringt deutliche Vorteile gegenüber herkömmlichen Verfahren zur Herstellung von Acetalen und Ketalen, insbesondere dadurch, dass bei relativ niedrigen Temperaturen hohe Reaktionsgeschwindigkeiten und Selektivitäten bei der Umsetzung erreichbar sind. Der Zufuhrstrom kann direkt und wahlweise flüssig oder als Dampf dem Membranmodul zugeführt werden.

Die Entfernung des Wassers ist für den Reaktionsfortschritt und damit für den raschen Ablauf der Umsetzung unbedingt erforderlich. Bei erhöhtem Fluss an einem üblichen hydrophilen Membrantyp werden neben Wasser auch größere Mengen Alkohol - insbesondere Methanol - abgetrennt, so dass besonders gegen Ende der Reaktion das im Reaktor verbleibende Produkt bereits einen sehr hohen Wertproduktanteil hat. Bei Einsatz von homogen verteilten Säuren als Katalysator ist vor der letzten Membrankaskade eine destillative oder extraktive Abtrennung der Säure erforderlich, um das gewünschte Acetal oder Ketal in hochreiner Form zu isolieren. Oft genügt in diesem Fall bereits eine destillative Aufarbeitung mit geringem Trennaufwand an einem Dünnschicht- oder Kurzwegverdampfer.

### Beispiel:

In einem Pervaporationsmodul für Versuche im Dampfpermeationsbetrieb bestehend aus einem Reaktionsbehälter mit einem Volumen von 1500 ml und einem Membranteil mit einer Membranfläche von ca. 0,1 m² wird eine Mischung aus 348 g Aceton, 576 g Methanol und 5 g Neodecansäure (Versatic 10 der Fa. Resolution) vorgelegt. Unter gutem Rühren wird die Mischung auf 60 °C erhitzt. Der gesättigte Dampf wird vom oberen Ende des Reaktionsbehälters zum oberen Ende der Membranzelle geführt und strömt an der Membran von oben nach unten vorbei. In Fließrichtung wird der Retentatdampf in einem Kühler kondensiert und mit Hilfe einer Kreislaufpumpe dem Reaktionsbehälter wieder zugeführt. Das die Membran passierende Permeat wird gesammelt und im Permeatkondensator kondensiert. Auf der Permeatseite wird ein Druck von 2 bis 10 mbar absolut angewendet. Das Kondensat besteht aus Wasser und Methanol in einem Verhältnis von ca. 1 : 0,5. Unter diesen Bedingungen wird bei der Entwässerung ein Fluss von ca. 1 kg/m²×h mit Hilfe einer Membran der Fa. SULZER CHEMTECH des Typs PERVAP 2210 oder 2510 erzielt. Der Umsatz zu Dimethoxypropan in der ersten Stufe beträgt ca. 55 %. Der Retentatstrom - bestehend aus 343 g Dimethoxypropan, 245 g Methanol und 156 g Aceton - wird absatzweise oder kontinuierlich einer weiteren Stufe eines Membranmoduls zugeführt. Zu 744 g dieser Retentatmischung aus der ersten Stufe werden 120 g Methanol hinzugeführt und erneut der Behandlung unter vergleichbaren Bedingungen wie in der ersten Stufe unterzogen. Als Permeat werden nun 168 g einer Mischung bestehend aus Methanol und Wasser in einem Verhältnis von 1 : 3 entnommen. Das Retentat enthält 618 g Dimethoxypropan, ca. 1 g Aceton und 295 g Methanol. Der Umsatz zu Dimethyoxypropan beträgt ca. 99 %. Diese Mischung wird nun einer weiteren Stufe zur Behandlung in einer Membrananlage zugeführt, wobei an einem anderen Membrantyp der Fa. SULZER CHEMTECH PERVAP 2256 unter einem Membranfluss von ca. 6 kg/m²×h aus diesem Zufuhrgemisch 290 g Methanol entfernt werden.
Das Retentat enthält nun eine Mischung aus 616 g Dimethoxypropan und 4 g Methanol, womit das Zielprodukt im Retentat einen Gehalt von 98,4 % aufweist.

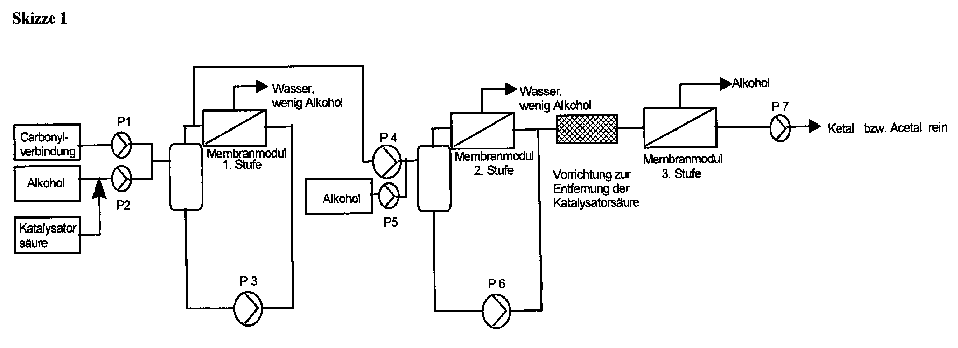

## Patentansprüche

1. Verfahren zur Herstellung von Acetalen oder Ketalen aus Carbonylverbindungen und Alkoholen in Gegenwart von sauren Katalysatoren,
**dadurch gekennzeichnet,**
**dass** Wasser und Alkohol aus dem Reaktionsgemisch durch eine mindestens zweistufig durchgeführte Pervaporation oder Dampfpermeation entfernt werden, wobei zusätzlicher Alkohol in den Retentatstrom mindestens einer der Stufen eingespeist wird und mindestens eine der Membranen in den Pervaporations- oder Dampfpermeationsstufen von den anderen Membranen in ihrem Selektivitätsverhalten bezüglich der Abtrennung von Wasser und Alkohol verschieden ist.

2. Verfahren nach Anspruch 1,
**dadurch gekennzeichnet,**
**dass** die Einspeisung des zusätzlichen Alkohols nach der ersten Pervaporations- oder Dampfpermeations-Stufe erfolgt.

3. Verfahren nach Anspruch 1 oder 2,
**dadurch gekennzeichnet,**
**dass** die zusätzlich eingespeiste Menge an Alkohol 10 - 500 Mol-% bezogen auf die eingesetzte Carbonylverbindung beträgt.

4. Verfahren nach Anspruch 1,
**dadurch gekennzeichnet,**
**dass** die Carbonylverbindung ein Aldehyd der allgemeinen Formel RCHO oder ein Keton der allgemeinen Formel R¹COR² ist, wobei R, R¹ und R² gleich oder verschieden sind und für einen Alkyl-, Alkenyl-, Cycloalkyl-, Aryl-, Alkylaryl- oder Aralkyl-Rest mit 1 - 15 Kohlenstoffatomen stehen und R¹ und R² auch zu einem Ring verbunden sein können und wobei der Alkylrest auch eine Acylfunktion mit 1 bis 8 Kohlenstoffatomen tragen kann.

5. Verfahren nach Anspruch 4,
**dadurch gekennzeichnet,**
**dass** als Carbonylverbindung Formaldehyd, Acetaldehyd, Propionaldehyd, n- und iso-Butyraldehyd, Acrolein, Benzaldehyd, Formamid, N,N-Dimethylformamid, Aceton, Methylethylketon, Acetophenon, Cyclohexanon, Cyclododecanon, Cyclodecandion oder Acetessigsäureester eingesetzt wird.

6. Verfahren nach Anspruch 1,
**dadurch gekennzeichnet,**
**dass** der Alkohol ein einwertiger oder mehrwertiger, unverzweigter oder verzweigter, gesättigter, ungesättigter oder aromatischer, Alkohol mit 1 bis 10 Kohlenstoffatomen ist.

7. Verfahren nach Anspruch 2,
**dadurch gekennzeichnet,**
**dass** als Alkohol Methanol, Ethanol, n-Propanol, iso-Propanol, Allylalkohol, Benzylalkohol, Ethylenglykol, Propandiol-1,3 oder Propandiol -1,2 eingesetzt wird.

8. Verfahren nach einem der vorhergehenden Ansprüche,
**dadurch gekennzeichnet,**
**dass** die sauren Katalysatoren in der der Reaktionsmischung gelöst, suspendiert oder als Feststoffpackung fixiert sind.

9. Verfahren nach Anspruch 8,
**dadurch gekennzeichnet,**
**dass** als saure Katalysatoren organische Carbonsäuren mit 1 bis 3 Carboxylgruppen mit 1 bis 12 Kohlenstoffatomen verwendet werden.

10. Verfahren nach Anspruch 8,
**dadurch gekennzeichnet,**
**dass** als saurer Katalysator Ameisensäure, Essigsäure, Oxalsäure, 2-Ethylhexansäure, Neooctansäure, Neononansäure, Neodecansäure, Methansulfonsäure oder p-Toluolsulfonsäure verwendet wird.

11. Verfahren nach einem der vorhergehenden Ansprüche,
**dadurch gekennzeichnet,**
**dass** der Katalysator ein Feststoff ist und in einer Packung angeordnet ist oder in einem Nebenstrom oder im Eduktgemisch suspendiert ist.

12. Verfahren nach einem der vorhergehenden Ansprüche,
**dadurch gekennzeichnet,**
**dass** die Temperaturen im Reaktor - 10 bis + 200 °C, bevorzugt + 20 bis + 150 °C, betragen.

13. Verfahren nach einem der vorhergehenden Ansprüche,
**dadurch gekennzeichnet,**
**dass** Drücke auf der Zufuhrseite der Pervaporations- oder Dampfpermeationsstufen von 0,1 bis 10 bar und auf der Permeatseite von 0,1 bis 1000 mbar angewendet werden.
